# EUROPÄISCHE PATENTANMELDUNG

(11) **EP 3 650 049 A1**
(43) Veröffentlichungstag der Anmeldung: **13.05.2020**
(21) Anmeldenummer: 19207961.4
(22) Anmeldetag: 08.11.2019
(51) Int. Cl.: A61L 2/02, A61L 2/18

(54) **VORRICHTUNG UND VERFAHREN ZUR HERSTELLUNG EINES FLUIDS ZUM VERSORGEN EINES VERBRAUCHERS**

(30) Priorität: 08.11.2018 DE 102018128000
(71) Anmelder: KRONES AG, 93073 Neutraubling (DE)
(72) Erfinder: Schafaczek, Bernd, Neutraubling (DE); Soellner, Juergen, Neutraubling (DE)
(74) Vertreter: Nordmeyer, Philipp Werner

(57) **Zusammenfassung**

Die vorliegende Erfindung betrifft eine Vorrichtung (1) zum Herstellung eines Fluids, bevorzugt eines Sperr-, Reinigungs- und/oder Desinfektionsfluids zum Versorgen eines Verbrauchers, bevorzugt eines Wasserschlosses (6) zur gasdichten Abdichtung eines Isolators einer Getränkeabfüllanlage, umfassend eine Prozesswasserzufuhr (2) zur Zufuhr von Prozesswasser, einen Filter (3) zum Sterilfiltern des zugeführten Prozesswassers und eine Desinfektionsmittelzufuhr (4) zur Zufuhr von Desinfektionsmittel zu dem Prozesswasser, wobei der Filter (3) stromabwärts der Zufuhr des Desinfektionsmittels zu dem Prozesswasser angeordnet ist, sowie ein entsprechendes Verfahren.

## Beschreibung

### Technisches Gebiet

Die vorliegende Erfindung betrifft eine Vorrichtung und ein Verfahren zur Herstellung eines Fluids, bevorzugt eines Sperr-, Reinigungs- und/oder Desinfektionsfluids zum Versorgen eines Verbrauchers, bevorzugt eines Wasserschlosses zur gasdichten Abdichtung eines Isolators einer Getränkeabfüllanlage.

### Stand der Technik

Es ist bekannt, bei Anwendungen, die hohe Anforderungen an die Hygiene stellen, beispielsweise das Abfüllen von Getränken, zumindest Teile der Anlage, beispielsweise einer Getränkeabfüllanlage, einzuhausen und dadurch Räume zur Verfügung zu stellen, deren Inneres von der äußeren Umgebung getrennt ist. In diesen Räumen kann eine gegenüber der äußeren Umgebung abgegrenzte Atmosphäre bereitgestellt werden, die beispielsweise eine verringerte mikrobiologische Belastung aufweist.

Bei Getränkeabfüllanlagen werden diese Reinräume beispielsweise durch die Bereitstellung eines sogenannten Isolators ermöglicht, in deren Isolatorinnenräumen eine kontrollierte Atmosphäre bereitgestellt wird. Dazu wird in dem Isolatorinnenraum typischer Weise ein leichter Überdruck gegenüber der Umgebung bereitgestellt, so dass ein Eindringen von Schmutz, Staub und mikrobiologischer Belastung von außen reduziert wird. Die Luft, die in den Isolator eingebracht wird, wird dabei üblicher Weise über Filteranlagen einer vorgegebenen Reinheitsstufe gereinigt, so dass die Luft im Isolator entsprechend eine vorgegebene Reinheitsstufe aufweist.

Isolatoren in Getränkeabfüllanlagen weisen oftmals starr angeordnete Komponenten, etwa eine Isolatorunterseite, und gegenüber diesen sich bewegende Komponenten, beispielsweise eine rotierende Isolatoroberseite - beispielsweise ein Dach eines Füllerkarussells - auf.

Um sicherzustellen, dass zwischen den stehenden Komponenten und den sich relativ dazu bewegenden Komponenten eine gasdichte Abdichtung vorliegt, ist es bekannt, sogenannte Wasserschlösser einzusetzen, insbesondere bei der Abgrenzung von drehenden und stehenden Teilen bei Rundläufermaschinen für die kaltaseptische Abfüllung. Bei einem solchen Wasserschloss handelt es sich um eine üblicherweise an der stehenden Komponente angeordnete Rinne, in die ein üblicher Weise an der drehenden Komponente angeordnetes Schwert eingreift. Die Wasserschlösser werden dabei mit einem Fluid befüllt, welches sich in der Regel aus sterilfiltriertem Wasser und einem Desinfektionsmittel zusammensetzt. Die Konzentration des Desinfektionsmittels wird dabei so gewählt, dass das Fluid eine bakterizide Wirkung entfalten kann. Das Schwert des Wasserschlosses taucht dann derart in das sich in der Rinne befindliche Fluid ein, dass eine zuverlässige und im Wesentlichen gasdichte Abdichtung erreicht wird.

Zur Herstellung eines solchen Fluides ist es bekannt, Prozesswasser und Desinfektionsmittel in einem Batchverfahren auszumischen. Dabei wird das Prozesswasser durch einen Sterilfilter filtriert und nach dem Filtern in einen Vorratsbehälter gegeben. In diesem Vorratsbehälter wird das gefilterte Prozesswasser mit dem Desinfektionsmittel versetzt und durchmischt, sodass ein Batch an Fluid in dem Tank vorliegt. Aus diesem Vorratsbehälter fließt das dann fertige Fluid nach Öffnung eines entsprechenden Ventils in die Rinne eines Wasserschlosses, um die Rinne auf die gewünschte Füllhöhe mit dem Fluid aufzufüllen.

Die Sterilisation der Prozesswasser führenden Leitung und insbesondere des Sterilfilters erfolgt, indem die Prozesswasserzufuhr geschlossen wird und über eine Sterildampf-Nebenleitung Sterildampf durch die Leitung und den Sterilfilter hindurch geleitet wird.

Mithin ist ein Dampfanschluss mit einer entsprechenden Drainagevorrichtung in der Leitung erforderlich, um eine Dampfsterilisation der Leitung und des Filters sowie eine Abfuhr des zur Sterilisierung verwendeten Dampfes zu gewährleisten. Die thermische Sterilisation des Sterilfilters resultiert darin, dass der Sterilfilter periodisch einer hohen thermischen Belastung ausgesetzt ist, wodurch dessen Lebensdauer reduziert wird.

Fernerhin sind für die Sterilisationsintervalle zusätzliche Prozesszeiten erforderlich, welche sich aufgrund der benötigten Heißhaltezeit der thermischen Sterilisation und durch den batchweisen Ansatz des Mediums ergeben.

### Darstellung der Erfindung

Ausgehend von dem bekannten Stand der Technik ist es eine Aufgabe der vorliegenden Erfindung, eine verbesserte Vorrichtung zur Herstellung eines Fluids, bevorzugt eines Sperr-, Reinigungs- und/oder Desinfektionsfluids zum Versorgen eines Verbrauchers, bevorzugt eines Wasserschlosses zur gasdichten Abdichtung eines Isolators einer Getränkeabfüllanlage, sowie ein entsprechendes Verfahren bereitzustellen.

Die Aufgabe wird durch eine Vorrichtung zur Herstellung eines Fluids, bevorzugt eines Sperr-, Reinigungs- und/oder Desinfektionsfluids zum Versorgen eines Verbrauchers, bevorzugt eines Wasserschlosses zur gasdichten Abdichtung eines Isolators einer Getränkeabfüllanlage, mit den Merkmalen des Anspruchs 1 gelöst. Vorteilhafte Weiterbildungen ergeben sich aus den Unteransprüchen, der Beschreibung und den beigefügten Figuren.

Entsprechend wird eine Vorrichtung zur Herstellung eines Fluids, bevorzugt eines Sperr-, Reinigungs- und/oder Desinfektionsfluids zum Versorgen eines Verbrauchers, bevorzugt eines Wasserschlosses zur gasdichten Abdichtung eines Isolators einer Getränkeabfüllanlage, vorgeschlagen, umfassend eine Prozesswasserzufuhr zur Zufuhr von Prozesswasser, einen Filter zum Sterilfiltern des zugeführten Prozesswassers und eine Desinfektionsmittelzufuhr zur Zufuhr von Desinfektionsmittel zu dem Prozesswasser. Erfindungsgemäß ist der Filter stromabwärts der Zufuhr des Desinfektionsmittels zu dem Prozesswasser angeordnet.

Dadurch, dass der Filter stromabwärts der Zufuhr des Desinfektionsmittels zu dem Prozesswasser angeordnet ist, kann auf eine zum Stand der Technik beschriebene thermische Sterilisation der Leitung sowie des Filters mittels Dampf verzichtet werden, da das Desinfektionsmittel bereits vor der Filtration des Prozesswassers zudosiert worden ist und dadurch durch die sterilisiere Wirkung des Desinfektionsmittels auf die Leitung und den Filter wirken kann. Mit anderen Worten kann dadurch eine Eigensterilisation der Leitung und des Filters durch das Prozesswasser-Desinfektionsmittel-Gemisch erfolgen.

Die Leitung und der Filter sind dabei bevorzugt stets oder nach einer vorgegebenen Einstellzeit mit dem Gemisch gefüllt, sodass eine ausreichende Einwirkzeit gewährleistet ist, oder der Filter und die Leitung können sogar im Wesentlichen permanent mit dem Gemisch gefüllt sein, sodass eine im Wesentlichen permanente Eigensterilisation erfolgt.

Während der Rüstzeiten einer die Vorrichtung aufweisenden Anlage verbleibt das Prozesswasser-Desinfektionsmittel-Gemisch in der Leitung und dem Filter, sodass auch dann eine im Wesentlichen permanente Sterilisation gewährleistet ist.

Ferner ist es nicht mehr erforderlich, gefiltertes Prozesswasser und Desinfektionsmittel in einem Vorratsbehälter batchweise vorzumischen. Durch den Wegfall dieser Komponenten und der thermischen Sterilisation mittels Sterildampf verringert sich die Anlagenkomplexität und die erforderlichen Prozesszeiten werden verkürzt.

Gemäß einer weiteren bevorzugten Ausführungsform ist eine Leitung vorgesehen, in welcher das Prozesswasser geführt wird, wobei die Desinfektionsmittelzufuhr das Desinfektionsmittel direkt in die Leitung zudosiert. Mit anderen Worten erfolgt eine sogenannte Inline-Dosierung, bei der das Desinfektionsmittel direkt in den Prozesswasserstrom zudosiert wird. Ein batchweises Ausmischen und Vorhalten des Gemischs ist mithin nicht mehr erforderlich. Folglich können die hierfür benötigten Komponenten weggelassen werden, sodass eine derart ausgebildete Vorrichtung einen besonders kompakten Aufbau aufweist und das Bereitstellen des Gemischs bedarfsgerecht stattfinden kann.

Um ein im Wesentlichen gleich bleibendes Mischverhältnis von Prozesswasser und Desinfektionsmittel zu erzielen, kann gemäß einer weiteren bevorzugten Ausführungsform in der Prozesswasserzuführleitung ein auf das Prozesswasser wirkender erster statischer Druck einstellbar sein und in der Desinfektionsmittelzufuhr ein auf das Desinfektionsmittel wirkender zweiter statischer Druck einstellbar sein, wobei das Mengenverhältnis von Prozesswasser zu Desinfektionsmittel über das Verhältnis von erstem statischen Druck zu zweitem statischen Druck eingestellt werden kann. Für eine Änderung des Mischverhältnisses kann folglich in einfacher Weise der erste statische Druck und/oder der zweite statische Druck durch Beaufschlagen eines Vorlagebehälters oder eines Prozesswasserreservoirs mit einem Zusatzdruck - beispielsweise durch Beaufschlagen mit einem Druckgas - angepasst werden.

Für die Bereitstellung des Desinfektionsmittels kann die Desinfektionsmittelzufuhr einen Vorlagebehälter zum Vorhalten von Desinfektionsmittel und Druckbeaufschlagen des Desinfektionsmittels aufweisen.

Gemäß einer weiteren bevorzugten Ausführungsform ist in der Prozesswasserzuführleitung eine auswechselbare und/oder einstellbare Blende zum Vorgeben eines Prozesswasserstromes in der Prozesswasserzuführleitung vorgesehen. Über die Blende kann ein Volumenstrom des Prozesswassers in der Leitung eingestellt beziehungsweise vorgegeben werden. Über eine Änderung des Volumenstroms kann wiederum das Mischverhältnis zwischen Prozesswasser und Desinfektionsmittel verändert werden.

Wenn zum Zudosieren des Desinfektionsmittels zu dem Prozesswasser ein Proportional-Dosierventil vorgesehen ist, welches derart ausbildet, dass es dem Prozesswasser eine entsprechend eines Flusses des Prozesswassers durch das Proportional-Dosierventil einstellbare vorgegebene Menge an Desinfektionsmittel beimengt, kann ein besonders genaues Zudosieren beziehungsweise Beimengen von Desinfektionsmittel zu dem Prozesswasser erfolgen, da die Zudosierung stets volumenproportional zum Prozesswasserstrom erfolgt. Dadurch ist eine Überwachung des Desinfektionsmittelflusses zur Sicherstellung der korrekten Ausmischung ausreichend. Eine derart aufgebaute Vorrichtung weist mithin einen besonders einfachen Aufbau auf, wobei das Fluid mit besonders exakt eingestelltem Mischverhältnis bereitgestellt werden kann.

Gemäß einer weiteren bevorzugten Ausführungsform ist ein Drainageventil zum Abführen von gefiltertem Desinfektionsmittel-Prozesswassergemisch vorgesehen. Dadurch ist es möglich, etwa eine Probe des Fluids zu entnehmen und zu ermitteln, ob das Mischverhältnis einem gewünschten Mischverhältnis spricht. Zudem kann dadurch auf einfache Weise ein Vorlauf erfolgen, indem beispielsweise nicht korrekt vermengtes Fluid zu Beginn des Mischvorgangs abgeführt wird, bevor ein Speisen des Verbrauchers mit dem Fluid erfolgt. Durch den Vorlauf kann auch ein Durchströmen und Desinfizieren des Filters erreicht werden, bevor das Gemisch an den Verbraucher geleitet wird.

Dabei ist zu berücksichtigen, dass bei einer noch nicht erfolgten vollständigen Desinfizierung des Filters ein Verbrauch an Desinfektionsmittel für die Filterdesinfektion stattfinden kann. Entsprechend kann sich in einem Zustand, in dem die Desinfektion des Filters noch nicht abgeschlossen ist, das Mischverhältnis vor dem Erreichen der Verbraucher noch ändern. Daher ist es bevorzugt, mittels des Drainageventils mittels eines Vorlaufs eine Desinfektion des Filters zu erreichen, bevor die Verbraucher mit dem Gemisch versorgt werden. Der Vorlauf kann beispielsweise über ein Vorlaufvolumen, eine Vorlaufdauer oder eine Messung des tatsächlich am Drainageventil anstehenden Mischverhältnisses gesteuert werden. Nach dem Vorlauf sollte sichergestellt sein, dass das durch die tatsächliche Mischung bereitgestellte Gemisch dem Gemisch entspricht, das tatsächlich gewünscht ist und entsprechend dann dem Verbraucher zur Verfügung gestellt werden kann.

Um ein Strömen des fertig abgemischten Fluids zu dem Verbraucher zu ermöglichen oder zu verhindern, kann zwischen dem Verbraucher und dem mindestens einen Filter ein Absperrventil zum Öffnen und Sperren eines Fluidstroms zu dem Verbraucher angeordnet sein.

Gemäß einer weiteren bevorzugten Weiterführung ist der Verbraucher ein Wasserschloss zur gasdichten Abdichtung eines Isolators, eine Verschließerschmierung, ein Auslaufbad einer Behälterreinigungsmaschine, eine Injektorkühlstrecke, eine Erhaltungsdesinfektion einer Getränkeabfüllanlage, eine Oberflächenreinigung in einer Getränkeabfüllanlage, ein Rinser, eine Außenbehandlung eines Isolators sowie andere Verbraucher von Reinigungs- und/oder Desinfektionsfluid.

Eine besonders vorteilhafte Wirkung des Fluids kann erzielt werden, wenn der Mengenanteil von Desinfektionsmittel im Desinfektionsmittel-Prozesswassergemisch 0,01 - 10 Vol.-% beträgt. Bevorzugt beträgt der Mengenanteil von Desinfektionsmittel im Desinfektionsmittel-Prozesswassergemisch 1-10 Vol.-%, bevorzugt bei 2-5 Vol.-% und besonders bevorzugt bei etwa 3 Vol.-% beträgt, insbesondere, wenn das Desinfektionsmittel Wasserstoffperoxid (H₂O₂) und/oder einen vergleichbaren Stoff der ein vergleichbares Stoffgemisch aufweist, oder alternativ beträgt der Mengenanteil von Desinfektionsmittel im Desinfektionsmittel-Prozesswassergemisch bevorzugt 0,01 - 1 Vol.-%, bevorzugt 0,05 - 0,5 Vol.-%, besonders bevorzugt 0,1 bis 0,3 Vol.-%, insbesondere, wenn das Desinfektionsmittel Peressigsäure (PES) und/oder einen vergleichbaren Stoff oder ein vergleichbares Stoffgemisch aufweist.

In einer weiteren bevorzugten Ausführungsform weist das Desinfektionsmittel Wasserstoffperoxid (H₂O₂), Peressigsäure (PES) und/oder einen vergleichbaren Stoff oder ein vergleichbares Stoffgemisch auf. Der Begriff "vergleichbar" ist hier so zu verstehen, dass der Stoff beziehungsweise das Stoffgemisch eine vergleichbar desinfizierende beziehungsweise sterilisierende Wirkung aufweist, wie die vorgenannten Stoffe.

Gemäß einer weiter bevorzugten Ausführungsform sind mehrere Filter, bevorzugt zwei, drei, vier oder fünf Filter, stromabwärts der Zufuhr des Desinfektionsmittels angeordnet.

Die oben gestellte Aufgabe wird weiterhin durch ein Verfahren zur Herstellung eines Fluids, bevorzugt eines Sperr-, Reinigung- und/oder Desinfektion Fluids zum Versorgen eines Verbrauchers, bevorzugt eines Wasserschlosses zur gasdichten Abdichtung eines Isolators in einer Getränkeabfüllanlage mit den Merkmalen des Anspruchs 12 gelöst. Vorteilhafte Weiterbildungen des Verfahrens ergeben sich aus den Unteransprüchen sowie der vorliegenden Beschreibung und den Figuren.

Entsprechend wird ein Verfahren zur Herstellung eines Fluids, bevorzugt eines Sperr-, Reinigungs- und/oder Desinfektionsfluids zum Versorgen eines Verbrauchers, bevorzugt eines Wasserschlosses zur gasdichten Abdichtung eines Isolators einer Getränkeabfüllanlage, vorgeschlagen, umfassend das Bereitstellen von Prozesswasser als Basis des Reinigungs- und/oder Desinfektionsfluids, das Zuführen beziehungsweise Beimengen beziehungsweise Zudosieren einer vorgegebenen Menge an Desinfektionsmittel in das Prozesswasser, das Filtrieren des Desinfektionsmittel-Prozesswassergemisches mittels mindestens eines Filters zum Sterilfiltern, und das Zuführen des gefilterten Desinfektionsmittel-Prozesswassergemisches als Versorgungsfluid zu dem Verbraucher.

Durch das Verfahren können die hinsichtlich der Vorrichtung beschriebenen Vorteile und Wirkungen analog erzielt werden.

Gemäß einer weiteren bevorzugten Ausführungsform wird das Prozesswasser in einer Leitung geführt und das Desinfektionsmittel direkt in die Leitung eindosiert.

Zur genauen Einstellung der desinfizierenden beziehungsweise sterilisierenden Wirkung des fertiggemischten Fluids kann vor dem Zuführen des gefilterten Desinfektionsmittel-Prozesswassergemisches an den Verbraucher eine Probenentnahme erfolgen, bevorzugt an einem Drainageventil, wobei bevorzugt die Menge an beigemengtem Desinfektionsmittel basierend auf einer Auswertung der Probenentnahme vor dem Zuführen angepasst wird.

### Kurze Beschreibung der Figuren

Bevorzugte weitere Ausführungsformen der Erfindung werden durch die nachfolgende Beschreibung der Figuren näher erläutert. Dabei zeigen:
- Figur 1: schematisch einen Schaltplan einer Vorrichtung zur Herstellung eines Fluids zum Versorgen eines Verbrauchers gemäß einer ersten Ausführungsform; und
- Figur 2: schematisch einen Schaltplan einer Vorrichtung zum Herstellen eines Fluids zum Versorgen eines Verbrauchers gemäß einer zweiten Ausführungsform.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

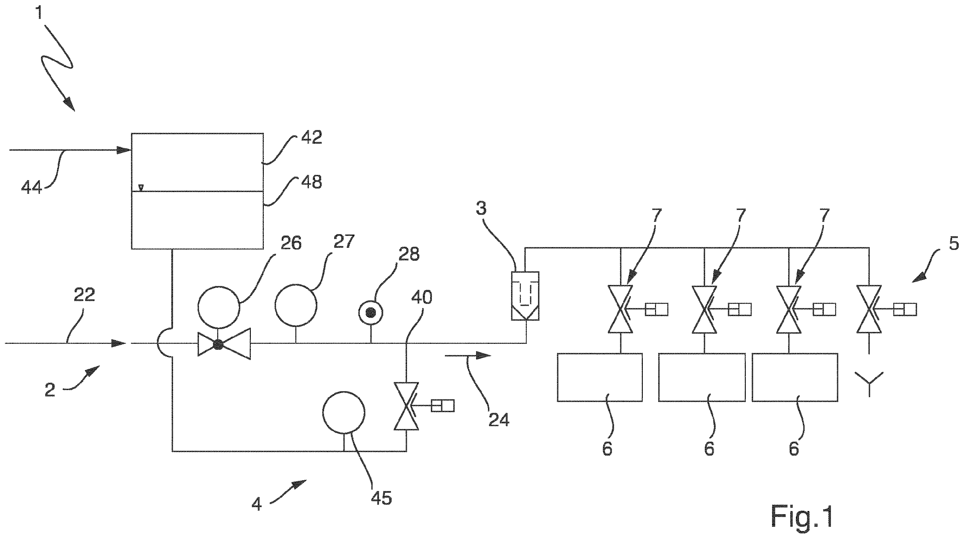

In Figur 1 ist schematisch ein Schaltplan einer Vorrichtung 1 zur Herstellung eines Fluids zum Versorgen einer Mehrzahl von Verbrauchern gemäß einer ersten Ausführungsform gezeigt. Die Verbraucher sind vorliegend als Wasserschloss 6 eines Isolators in einer Getränkeabfüllanlage ausgebildet.

Die Vorrichtung 1 umfasst eine Prozesswasserzufuhr 2, einen Filter 3 zum Sterilfiltern des zugeführten Prozesswassers, und eine Desinfektionsmittelzufuhr 4 zur Zufuhr von Desinfektionsmittel zu dem Prozesswasser.

Das Prozesswasser wird, wie mittels des Bezugszeichens 22 angedeutet, in eine Leitung 20 der Prozesswasserzufuhr 2 eingeleitet. An einer vorgegebenen Stelle 40 der Leitung 20 erfolgt die Zufuhr des Desinfektionsmittels zu dem Prozesswasser in die Leitung 20. Nach dem Beimengen des Desinfektionsmittels wird das Desinfektionsmittel-Prozesswasser-Gemisch an den Filter 3 weitergeleitet, welcher sich in einer Strömungsrichtung 24 gesehen stromabwärts der Zufuhr des Desinfektionsmittels zu dem Prozesswasser befindet.

Nach Durchlaufen des Filters 3 kann das fertige Fluid über Absperrventile 7 den einzelnen Wasserschlössern 6 zugeführt und/oder über ein Drainageventil 5 abgelassen werden.

Das Mischverhältnis von Desinfektionsmittel und Prozesswasser wird gemäß dieser Ausführungsform über das Verhältnis von einem ersten statischen Druck, welcher auf das Prozesswasser wirkt, und einem zweiten statischen Druck, welcher auf das Desinfektionsmittel wirkt, eingestellt beziehungsweise vorgegeben. Zum Einstellen des ersten statischen Drucks ist in der Leitung 20 stromaufwärts der Zufuhr des Desinfektionsmittels eine als Druckregeleinrichtung ausgebildete Druckeinstellung 26 angeordnet. Diese ist vorliegend beispielsweise derart eingestellt, sodass der erste statische Druck 3 bar entspricht.

Die Desinfektionsmittelzufuhr 4 weist einen Vorlagebehälter 42 auf, in welchem das Desinfektionsmittel 48 vorgehalten wird. Ferner wird das Desinfektionsmittel 48 in dem Vorlagebehälter 42 mit Druckluft 44 beaufschlagt, welche vorliegend mit einem statischen Druck von 4 bar auf das Desinfektionsmittel wirkt, sodass der zweite statische Druck entsprechend 4 bar ist.

Die korrekte Ausmischung beziehungsweise das korrekte Mischverhältnis, welches vorliegend mit 3 Vol.-% vorgegeben ist, wird durch eine Überwachung des Prozesswasserdrucks und des Desinfektionsmitteldurchflusses sichergestellt. Hierfür ist der Druckeinstellung 26 stromabwärts nachgelagert ein Druckmesser 27 zum Messen des statischen Drucks und dem Vorlagebehälter 42 nachgelagert ein Durchflussmesser 45 angeordnet.

Ferner ist in der Leitung 20 eine einstellbare Blende 28 vorgesehen, mittels welcher ein Volumenstrom des Prozesswassers einstellbar ist. Darüber lässt sich folglich bei konstantem ersten statischen Druck und konstantem zweiten statischen Druck das Mischverhältnis von Desinfektionsmittel und Prozesswasser einstellen.

Vorliegend wird als Desinfektionsmittel Wasserstoffperoxid verwendet. Alternativ kann auch ein anderes Desinfektionsmittel, beispielsweise Peressigsäure oder ein vergleichbares Desinfektionsmittel verwendet werden.

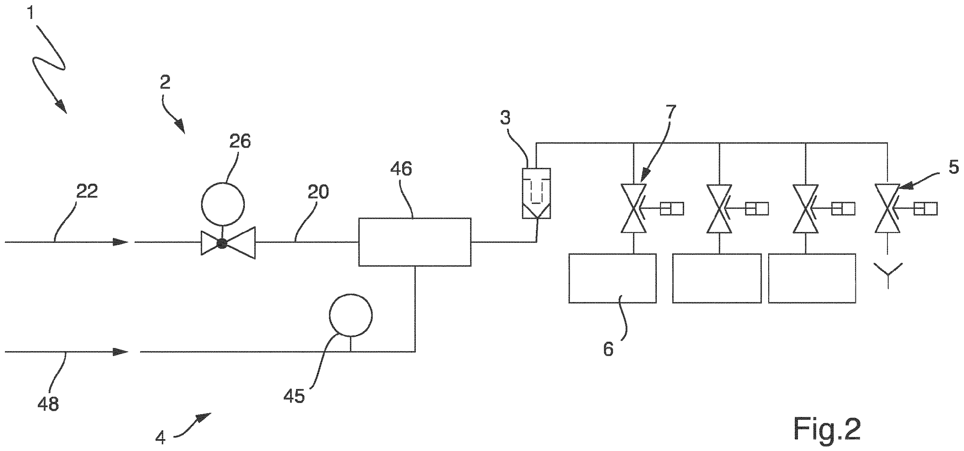

Figur 2 zeigt schematisch einen Schaltplan einer Vorrichtung 1 zum Herstellen eines Fluids zum Versorgen eines Verbrauchers gemäß einer zweiten Ausführungsform. Der Aufbau der Vorrichtung 1 entspricht im Wesentlichen jener der in Figur 1 gezeigten. Zum Zudosieren von Desinfektionsmittel zu dem Prozesswasser ist in dieser Ausführungsform jedoch ein Proportional-Dosierventil 46 vorgesehen, welches dem Prozesswasser entsprechend seines Flusses eine vorgegebene Menge an Desinfektionsmittel beimengt. Bei dieser Ausführungsform kann auf einen Vorlagebehälter für das Desinfektionsmittel verzichtet werden. Zudem ist es ausreichend, zur Sicherstellung der korrekten Ausmischung beziehungsweise des korrekten Mischverhältnisses den Durchfluss des Desinfektionsmittels durch den Durchflussmesser 45 zu überwachen.

Das Beimengen des Desinfektionsmittels zu dem Prozesswasser erfolgt mithin wiederum "inline". Im Anschluss an das Zuführen des Desinfektionsmittels zu dem Prozesswasser gelangt das Desinfektionsmittel-Prozesswasser-Gemisch zu dem Filter 3, in welchem es sterilgefiltert wird und somit das fertige Fluid erhalten wird, welches nun den Wasserschlössern 6 als Versorgungsfluid zugeführt werden kann.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezuqszeichenliste

- 1: Vorrichtung
- 2: Prozesswasserzufuhr
- 20: Leitung
- 22: Prozesswasser
- 24: Strömungsrichtung
- 26: Druckeinstellung
- 27: Druckmesser
- 28: Blende
- 3: Filter
- 4: Desinfektionsmittelzufuhr
- 40: vorgegebene Stelle
- 42: Vorlagebehälter
- 44: Druckluft
- 45: Durchflussmesser
- 46: Proportional-Dosierventil
- 48: Desinfektionsmittel
- 5: Drainageventil
- 6: Wasserschloss
- 7: Absperrventil

## Patentansprüche

1. Vorrichtung (1) zum Herstellung eines Fluids, bevorzugt eines Sperr-, Reinigungs- und/oder Desinfektionsfluids zum Versorgen eines Verbrauchers, bevorzugt eines Wasserschlosses (6) zur gasdichten Abdichtung eines Isolators einer Getränkeabfüllanlage, umfassend eine Prozesswasserzufuhr (2) zur Zufuhr von Prozesswasser, einen Filter (3) zum Sterilfiltern des zugeführten Prozesswassers und eine Desinfektionsmittelzufuhr (4) zur Zufuhr von Desinfektionsmittel zu dem Prozesswasser,
**dadurch gekennzeichnet, dass**
der Filter (3) stromabwärts der Zufuhr des Desinfektionsmittels zu dem Prozesswasser angeordnet ist.

2. Vorrichtung (1) gemäß Anspruch 1, **dadurch gekennzeichnet, dass** eine Leitung (20) vorgesehen ist, in welcher das Prozesswasser geführt wird, wobei die Desinfektionsmittelzufuhr (4) das Desinfektionsmittel direkt in die Leitung (20) eindosiert.

3. Vorrichtung (1) gemäß Anspruch 1 oder 2, **dadurch gekennzeichnet, dass** in der Leitung (20) ein auf das Prozesswasser wirkender erster statischer Druck einstellbar ist und in der Desinfektionsmittelzufuhr (4) ein auf das Desinfektionsmittel wirkender zweiter statischer Druck einstellbar ist, wobei das Mengenverhältnis von Prozesswasser zu Desinfektionsmittel über das Verhältnis von erstem statischen Druck zu zweitem statischen Druck einstellbar ist.

4. Vorrichtung (1) gemäß einem der vorstehenden Ansprüche, **dadurch gekennzeichnet, dass** die Desinfektionsmittelzufuhr (4) einen Vorlagebehälter (42) zum Vorhalten von Desinfektionsmittel (48) und zum Druckbeaufschlagen des Desinfektionsmittels (48) aufweist.

5. Vorrichtung (1) gemäß einem der vorstehenden Ansprüche, **dadurch gekennzeichnet, dass** in der Leitung (20) eine auswechselbare und/oder einstellbare Blende (28) zum Vorgeben eines Prozesswasserstromes in der Leitung (20) vorgesehen ist.

6. Vorrichtung (1) gemäß einem der vorstehenden Ansprüche, **gekennzeichnet durch** ein Proportional-Dosierventil (46), welches derart ausgebildet ist, dass es dem Prozesswasser eine entsprechend eines Flusses des Prozesswassers durch das Proportional-Dosierventil (46) vorgegebene Menge an Desinfektionsmittel beimengt.

7. Vorrichtung (1) gemäß einem der vorstehenden Ansprüche, **dadurch gekennzeichnet, dass** ein stromabwärts des Filters (3) angeordnetes Drainageventil (5) zum Abführen eines gefilterten Desinfektionsmittel-Prozesswassergemischs vorgesehen ist.

8. Vorrichtung (1) gemäß einem der vorstehenden Ansprüche, **dadurch gekennzeichnet, dass** zwischen dem Verbraucher und dem Filter (3) ein Absperrventil (7) zum Öffnen und Sperren eines Fluidstroms zu dem Verbraucher angeordnet ist.

9. Vorrichtung (1) gemäß einem der vorstehenden Ansprüche, **dadurch gekennzeichnet, dass** der Verbraucher ein Wasserschloss (6) zur gasdichten Abdichtung eines Isolators, eine Verschließerschmierung, ein Auslaufbad einer Behälterreinigungsanlage, eine Injektorkühlstrecke, eine Erhaltungsdesinfektion einer Getränkeabfüllanlage, eine Oberflächenreinigung in einer Getränkeabfüllanlage, ein Rinser, eine Außenbehandlung eines Isolators sowie andere Verbraucher von Reinigungs- und/oder Desinfektionsfluid ist.

10. Vorrichtung (1) gemäß einem der vorstehenden Ansprüche, **dadurch gekennzeichnet, dass** der Mengenanteil von Desinfektionsmittel im Desinfektionsmittel-Prozesswassergemisch0,01 - 10 Vol.-% beträgt, wobei bevorzugt der Mengenanteil von Desinfektionsmittel im Desinfektionsmittel-Prozesswassergemisch 1-10 Vol.-%, bevorzugt bei 2-5 Vol.-% und besonders bevorzugt bei etwa 3 Vol.-% beträgt, insbesondere, wenn das Desinfektionsmittel Wasserstoffperoxid (H₂O₂) und/oder einen vergleichbaren Stoff oder ein vergleichbares Stoffgemisch aufweist, oder bevorzugt der Mengenanteil von Desinfektionsmittel im Desinfektionsmittel-Prozesswassergemisch 0,01 - 1 Vol.-%, bevorzugt 0,05 - 0,5 Vol.-%, besonders bevorzugt 0,1 bis 0,3 Vol.-% beträgt, insbesondere, wenn das wenn das Desinfektionsmittel Peressigsäure (PES) und/oder einen vergleichbaren Stoff oder ein vergleichbares Stoffgemisch aufweist.

11. Vorrichtung gemäß einem der vorstehenden Ansprüche, **dadurch gekennzeichnet, dass** das Desinfektionsmittel Wasserstoffperoxid (H₂O₂), Peressigsäure (PES) und/oder einen vergleichbaren Stoff oder ein vergleichbares Stoffgemisch aufweist.

12. Verfahren zum Versorgen eines Verbrauchers, bevorzugt eines Wasserschlosses (6) zur gasdichten Abdichtung eines Isolators einer Getränkeabfüllanlage, mit einem Versorgungsfluid, bevorzugt einem Sperr-, Reinigungs- und/oder Desinfektionsfluid, umfassend das
- Bereitstellen von Prozesswasser als Basis des Sperr-, Reinigungs- und/oder Desinfektionsfluids;
- Beimengen einer vorgegebenen Menge an Desinfektionsmittel zu dem Prozesswasser;
- Sterilfiltrieren des Desinfektionsmittel-Prozesswassergemisches mittels eines Filters (3); und
- Zuführen des gefilterten Desinfektionsmittel-Prozesswassergemisches als Versorgungsfluid zu dem Verbraucher.

13. Verfahren gemäß dem vorstehenden Anspruch, **dadurch gekennzeichnet, dass** das Prozesswasser in einer Leitung (20) geführt wird und das Desinfektionsmittel direkt in die Leitung (20) eindosiert wird.

14. Verfahren gemäß Anspruch 12 oder 13, **dadurch gekennzeichnet, dass** vor dem Zuführen des gefilterten Desinfektionsmittel-Prozesswassergemisches zu dem Verbraucher eine Probenentnahme erfolgt, bevorzugt an einem Drainageventil (5), wobei bevorzugt die Menge an beigemengtem Desinfektionsmittel basierend auf einer Auswertung der Probenentnahme vor dem Zuführen angepasst wird.
